# EUROPEAN PATENT SPECIFICATION

(11) **EP 1 190 039 B1**
(45) Date of publication and mention of the grant of the patent: **14.12.2005**
(21) Application number: 00948500.4
(22) Date of filing: 09.06.2000
(51) Int. Cl.: C12N 5/04, C12N 15/29, C12N 15/63, C12N 15/70, C12N 15/74, C12N 15/79, C12N 15/81, C12N 15/82, C12N 15/83, C12N 15/90, C12N 9/90

(54) **GENE ENCODING SHORT INTEGUMENTS AND USES THEREOF**
FüR SIN KODIERENDES GEN UND DESSEN VERWENDUNGEN
GENE CODANT POUR SIN ET SES UTILISATIONS

(30) Priority: 09.06.1999 US 138316 P
(43) Date of publication of application: 27.03.2002
(73) Proprietor: UNIVERSITY OF ROCHESTER, Rochester, NY 14627-0140 (US)
(72) Inventor: RAY, Animesh, San Diego, CA 92130 (US); GOLDEN, Teresa, Ann, Pensacola, FL 32501 (US)
(74) Representative: Jones, Stephen Anthony
(86) International application number: PCT/US2000/015800
(87) International publication number: WO 2000/075359

(56) References cited:
- JACOBSEN STEVEN E ET AL: "Disruption of an RNA helicase/RNAse III gene in Arabidopsis causes unregulated cell division in floral meristems." DEVELOPMENT (CAMBRIDGE), vol. 126, no. 23, December 1999 (1999-12), pages 5231-5243, XP002232718 ISSN: 0950-1991 -& DATABASE EMBL [Online] EBI; 24 October 1999 (1999-10-24) "Arabidopsis thaliana CAF protein mRNA" retrieved from EMBL Database accession no. AF187317 XP002232720
- GASSER C S ET AL: "Genetic analysis of ovule development." ANNUAL REVIEW OF PLANT PHYSIOLOGY AND PLANT MOLECULAR BIOLOGY, vol. 49, 1998, pages 1-24, XP002232719 1998 Annual Reviews Inc. P.O. Box 10139, 4139 El Camino Way, Palo Alto, California 94306, USA ISBN: 0-8243-0649-X
- DATABASE EMBL [Online] EBI; 6 December 2000 (2000-12-06) "Arabidopsis thaliana short integuments 1 (SIN1) mRNA" retrieved from EMBL Database accession no. AF292940 XP002232721
- RAY ET AL.: 'Short integument (SIN1), a gene required for ovule development in arabidopsis, also controls flowering time' DEVELOPMENT vol. 122, 1996, pages 2631 - 2638, XP002934621
- RAY ET AL.: 'Maternal effects of the short integument mutation on embryo development in arabidopsis' DEV. BIOL. vol. 180, 1996, pages 365 - 369, XP002934620
- LANG ET AL.: 'SIN1, a mutation affecting female fertility in arabidopsis, interacts with MOD1, its recessive modifier' GENETICS vol. 137, August 1994, pages 1101 - 1110, XP002934619
- ROBINSON-BEERS ET AL.: 'Ovule development in wild-type arabidopsis and two female-sterile mutants' PLANT CELL vol. 4, October 1992, pages 1237 - 1249, XP002934618
- REISER ET AL.: 'The ovule and the embryo sac' PLANT CELL vol. 5, October 1993, pages 1291 - 1301, XP002934617
- BAKER ET AL.: 'Interactions among genes regulating ovule development in arabidopsis thaliana' GENETICS vol. 145, April 1997, pages 1109 - 1124, XP002934616
- SCHNEITZ ET AL.: 'Dissection of sexual organ ontogenesis: A genetic analysis of ovule development in arabidopsis thaliana' DEVELOPMENT vol. 124, 1997, pages 1367 - 1376, XP002934615

### SEQUENCE LISTING

<110> University of Rochester
<120> GENE ENCODING SHORT INTEGUMENTS AND USES THEREOF
<130> 176/60582
<140>
   <141>
<150> 60/138,316
   <151> 1999-06-09
<160> 2
<170> Patent In Ver. 2.1
<210> 1
   <211> 6184
   <212> DNA
   <213> Arabidopsis thaliana
<400> 1
   gaagacgaag agagaaacag aacagagtag ggatcgatag accgtggaat ctcagaatca 60
   caaacacttt gcaaaagggt tttcaattcc tatttattta caaagaaatc atcaatagta 120
   gtggtctcta gggttttgct tgctcttctt cgtgacccct ttttacctgc aaacaacaac 180
   ttcaaaattg gcgtgtttcg tacggtctat ctaaccctaa tctgtcacaa aacactcttc 240
   ttctctcacc cctttttctg ggtttattca attctcgtgc ttttggttct gttttcttct 300
   ctggggattt ggttttcttg agtgagtttt tctcctcttt cttatgttct tgatttgatt 360
   attatataga attatggtaa tggaggatga gcctagagaa gccacaataa agccttctta 420
   ttggctagat gcttgcgagg acatctcttg tgatcttatc gatgatctcg tgtctgaatt 480
   tgatccttcc tctgttgctg tcaatgaatc cactgatgaa aacggcgtca tcaatgattt 540
   tttcggtggg attgatcaca ttttagatag tatcaagaac ggtggaggct taccaaacaa 600
   tggcgtttct gataccaatt ctcaaatcaa cgaggttact gtaactcctc aggttattgc 660
   taaggagaca gtgaaggaga atgggttgca aaagaatggc ggtaagagag acgaattctc 720
   gaaagaggaa ggagacaagg ataggaagag agctagggtt tgtagttatc agagtgaaag 780
   gagtaacctt tcaggtagag ggcatgttaa taattctagg gagggagata ggtttatgaa 840
   taggaaacgt actcgtaatt gggacgaggc gggtaacaat aagaagaaaa gggaatgtaa 900
   caattacaga agagatggta gagatagaga agttaggggt tattgggaga gggataaagt 960
   tggttccaat gagttggttt ataggtcagg gacttgggaa gctgatcatg aaagagatgt 1020
   taagaaagtg agtggtggaa accgcgaatg cgatgtcaag gcagaggaga acaagagtaa 1080
   gcctgaagaa cgtaaagaga aggttgtgga agagcaagca aggcgatacc agttggatgt 1140
   tcttgaacaa gctaaagcga aaaacacgat tgctttcctt gagaccggtg ctggaaagac 1200
   acttatcgcg attcttctta ttaaaagtgt tcataaggat ctgatgagcc agaacagaaa 1260
   aatgctctcg gtgttcttgg ttcccaaagt gcctttggtt tatcagcaag cagaagtgat 1320
   ccgtaatcaa acttgttttc aagttggaca ttattgtggt gagatgggac aggacttttg 1380
   ggattctcga aggtggcaac gagagtttga gtctaagcag gttctagtta tgacagcaca 1440
   aattctgttg aatatactga gacacagtat cattagaatg gaaacaattg atcttcttat 1500
   tctcgacgag tgtcaccacg ctgtcaagaa acatccatac tctttagtga tgtcagagtt 1560
   ttaccataca actcctaaag ataaaagacc tgccatcttt ggaatgactg cttcgcctgt 1620
   taatttaaag ggtgtttcaa gccaagtaga ttgtgcgata aagatacgta acctcgagac 1680
   caagttggat tctacggttt gtactataaa agatcgaaaa gaattagaga aacatgtgcc 1740
   tatgccttca gagatagtcg tcgagtatga caaagctgct actatgtggt ctcttcatga 1800
   gacaataaag caaatgattg cagctgttga agaagcggca caagcaagtt caaggaaaag 1860
   caagtggcaa tttatggggg ctagggatgc tggagcaaag gatgaattga gacaggttta 1920
   tggcgtctct gaaagaacgg agagcgatgg tgctgccaat ttgattcata aacttagagc 1980
   tatcaattat actcttgctg aattgggtca atggtgtgct tacaaggtgg gacaatcatt 2040
   cttgtctgct ttgcaaagtg atgagagggt gaatttccaa gtcgacgtga agtttcaaga 2100
   atcatacctc agtgaggtgg tgtcactctt gcaatgtgag cttctggaag gcgctgctgc 2160
   tgaaaaagtc gcggcggaag ttggcaaacc agaaaatggt aatgcacatg acgagatgga 2220
   ggagggagag ctccctgatg atcctgtggt ctcgggaggg gagcacgttg atgaagtaat 2280
   aggcgccgca gtggctgatg ggaaagttac tccaaaagta caatcattga tcaaactact 2340
   cctcaaatat cagcacacag ctgattttcg agctattgtt ttcgttgaga gggtggttgc 2400
   tgctttggtt cttcctaagg tttttgcgga gctgccttcg cttagtttta tacggtgtgc 2460
   cagcatgatt ggacacaata acagccagga gatgaaatca tctcaaatgc aggatacaat 2520
   ttccaaattc cgagatgggc atgtgacact gttagttgcc acaagcgttg ctgaggaagg 2580
   acttgatatt aggcaatgta acgttgttat gcgtttcgac cttgcaaaga cggtgctggc 2640
   atacattcag tctcgtggcc gggcaagaaa gcctggatca gactacatac tcatggttga 2700
   gagaggaaat gtatctcacg cagcgttcct aaggaatgct aggaacagtg aggagacact 2760
   tcgaaaagaa gcaatagaaa ggactgatct tagtcatctc aaagatacat cgagattaat 2820
   ctcaattgat gctgtgcctg gtacagttta taaggtggag gcaactggtg ccatggttag 2880
   cttgaattcc gcggttggtc ttgtacattt ctactgctct cagcttcctg gtgacaggta 2940
   tgcaatcctt cgtcctgagt ttagcatgga gaagcatgaa aagcctgggg gccacacgga 3000
   atattcatgt aggcttcagc ttccttgcaa tgcaccgttt gaaatacttg agggtcctgt 3060
   ttgcagttca atgcgtcttg cacaacaggc tgtatgttta gctgcttgca agaaactgca 3120
   tgagatgggt gcatttaccg atatgctatt accggacaaa ggaagtggtc aagacgctga 3180
   gaaggctgac caagatgatg aaggtgagcc tgttcctgga actgctagac atagagagtt 3240
   ctatcctgaa ggtgtggcgg atgtacttaa gggagaatgg gtttcatctg gaaaggaagt 3300
   ttgtgagagc tcaaagctat tccatttata catgtataat gtcagatgtg tagattttgg 3360
   ctcttcaaaa gatccattcc taagcgaagt ttcagagttc gcgattcttt ttggcaatga 3420
   gctggatgca gaggtattat cgatgtctat ggatctttat gttgctcggg ccatgatcac 3480
   taaagcatct cttgctttca agggatcact tgatattaca gaaaaccagc tatcatctct 3540
   aaaaaagttt catgtgaggt taatgagtat cgtgttggat gttgatgttg aaccctccac 3600
   gacaccatgg gatcctgcaa aggcctacct gtttgtccct gttactgaca atacgtctat 3660
   ggaacccata aaagggatca actgggaatt ggttgaaaag attacgaaaa ccacagcgtg 3720
   ggacaaccct cttcagagag ctcgtcccga tgtatatctc gggactaatg agagaactct 3780
   tggtggggac agaagggaat atgggtttgg taaacttcgt cacaacattg tatttgggca 3840
   gaaatctcac ccaacttatg gtattagagg agctgttgca tccttcgatg ttgtgagagc 3900
   ttctggattg ttacctgtga gagatgcttt tgagaaggaa gtagaagagg atttatcaaa 3960
   aggaaaattg atgatggctg atgggtgcat ggttgcagaa gatcttattg ggaaaatagt 4020
   gacagccgca cattccggga agcggtttta cgtagattca atttgttatg acatgagtgc 4080
   agaaacatct ttccctagga aagagggata tcttggtccc ctagagtaca acacgtacgc 4140
   tgactattac aagcaaaagt atggagttga tttgaactgt aagcaacaac ctttgattaa 4200
   aggacgtggt gtttcgtatt gcaagaacct tctttctcct cggtttgaac agtcaggtga 4260
   atctgagaca gtccttgata agacatatta cgtgtttctt ccacctgaac tatgcgttgt 4320
   gcatccgctt tcgggttcac ttatccgagg tgctcagagg ttaccctcta taatgagaag 4380
   agttgagagc atgttactcg ctgttcaact caaaaatttg attagttatc ctattcccac 4440
   atcaaagatt cttgaagcct tgactgccgc ctcgtgccag gaaacgttct gctacgagag 4500
   agctgagctt ttaggagatg cgtatctaaa atgggttgtt agtcgttttc tgtttctcaa 4560
   gtatcctcaa aagcacgagg gtcagcttac aaggatgagg caacaaatgg ttagtaatat 4620
   ggttctttat cagtttgctc tggttaaagg gcttcagtca tatatccagg cggatcgatt 4680
   cgccccgtct aggtggtctg ctcctggtgt gcctccggtt ttcgacgagg acacaaaaga 4740
   tggaggatct tcgtttttcg atgaagagca aaaacctgtt tccgaggaaa acagcgatgt 4800
   gtttgaagat ggggagatgg aggatggtga actagagggt gatttgagtt cgtaccgagt 4860
   tttatctagc aaaacgttag ctgatgttgt tgaggctttg attggtgttt attacgtcga 4920
   agggggtaag attgcagcta atcatttgat gaaatggatt gggattcacg tggaggatga 4980
   tcctgatgaa gtcgatggaa cattgaaaaa tgttaatgtt ccagagagtg tgctcaagag 5040
   catcgacttt gttggtcttg agagagctct taaatatgag tttaaagaga aaggtcttct 5100
   tgttgaagct ataacacatg cttcaagacc atcttcaggt gtttcgtgtt accagagatt 5160
   ggaatttgtt ggtgacgcgg tcttggatca tctcatcaca agacatctat ttttcacata 5220
   cacaagcctt cctcctggtc ggttaacaga tcttcgagct gcagcggtta acaacgagaa 5280
   ttttgctcgc gttgcggtta aacataaact ccacttgtac cttcgtcacg gttcaagcgc 5340
   cctcgaaaaa cagattcggg aatttgtgaa ggaggttcaa accgagtcat cgaaaccggg 5400
   gtttaactct tttggtttgg gagactgcaa agcaccaaaa gttcttggag acattgttga 5460
   atctattgca ggtgctattt ttcttgatag tggaaaagat acaactgctg cttggaaggt 5520
   ttttcaacct ttgcttcagc ccatggtgac accagagaca cttccaatgc atccggtgcg 5580
   agagctacaa gagcggtgcc agcaacaagc agaagggtta gaatacaaag cgagtaggag 5640
   tggtaacaca gcgactgtgg aagttttcat cgacggtgtt caagttggag tagcgcaaaa 5700
   cccgcagaag aaaatggctc aaaagctagc tgcgaggaac gcacttgcag ctttgaaaga 5760
   gaaagaaata gcagaatcaa aggagaagca tatcaacaac ggtaatgcgg gagaggatca 5820
   aggcgagaat gagaatggga acaagaagaa tgggcatcag ccgtttacga gacaaacgtt 5880
   gaatgatatt tgtttgagga agaattggcc aatgccttct tacagatgtg tgaaagaagg 5940
   aggaccggct catgcaaaga gatttacgtt tggggtaaga gttaatacga gcgatagagg 6000
   atggaccgat gagtgtattg gcgagccaat gccgagtgtt aagaaagcta aggattcagc 6060
   tgcggttctt ctacttgagc ttttaaataa aactttttct tgattctttt actctcttca 6120
   acgagatgta gtcattacat tttaaacctt aaaaccatag tggttgtagt gttttaaaaa 6180
   aaaa 6184
<210> 2
   <211> 1909
   <212> PRT
   <213> Arabidopsis thaliana
<400> 2
   Met Val Met Glu Asp Glu Pro Arg Glu Ala Thr Ile Lys Pro Ser Tyr
   1 5 10 15
   Trp Leu Asp Ala Cys Glu Asp Ile Ser Cys Asp Leu Ile Asp Asp Leu
   20 25 30
   Val Ser Glu Phe Asp Pro Ser Ser Val Ala Val Asn Glu Ser Thr Asp
   35 40 45
   Glu Asn Gly Val Ile Asn Asp Phe Phe Gly Gly Ile Asp His Ile Leu
   50 55 60
   Asp Ser Ile Lys Asn Gly Gly Gly Leu Pro Asn Asn Gly Val Ser Asp
   65 70 75 80
   Thr Asn Ser Gln Ile Asn Glu Val Thr Val Thr Pro Gln Val Ile Ala
   85 90 95
   Lys Glu Thr Val Lys Glu Asn Gly Leu Gln Lys Asn Gly Gly Lys Arg
   100 105 110
   Asp Glu Phe Ser Lys Glu Glu Gly Asp Lys Asp Arg Lys Arg Ala Arg
   115 120 125
   Val Cys Ser Tyr Gln Ser Glu Arg Ser Asn Leu Ser Gly Arg Gly His
   130 135 140
   Val Asn Asn Ser Arg Glu Gly Asp Arg Phe Met Asn Arg Lys Arg Thr
   145 150 155 160
   Arg Asn Trp Asp Glu Ala Gly Asn Asn Lys Lys Lys Arg Glu Cys Asn
   165 170 175
   Asn Tyr Arg Arg Asp Gly Arg Asp Arg Glu Val Arg Gly Tyr Trp Glu
   180 185 190
   Arg Asp Lys Val Gly Ser Asn Glu Leu Val Tyr Arg Ser Gly Thr Trp
   195 200 205
   Glu Ala Asp His Glu Arg Asp Val Lys Lys Val Ser Gly Gly Asn Arg
   210 215 220
   Glu Cys Asp Val Lys Ala Glu Glu Asn Lys Ser Lys Pro Glu Glu Arg
   225 230 235 240
   Lys Glu Lys Val Val Glu Glu Gln Ala Arg Arg Tyr Gln Leu Asp Val
   245 250 255
   Leu Glu Gln Ala Lys Ala Lys Asn Thr Ile Ala Phe Leu Glu Thr Gly
   260 265 270
   Ala Gly Lys Thr Leu Ile Ala Ile Leu Leu Ile Lys Ser Val His Lys
   275 280 285
   Asp Leu Met Ser Gln Asn Arg Lys Met Leu Ser Val Phe Leu Val Pro
   290 295 300
   Lys Val Pro Leu Val Tyr Gln Gln Ala Glu Val Ile Arg Asn Gln Thr
   305 310 315 320
   Cys Phe Gln Val Gly His Tyr Cys Gly Glu Met Gly Gln Asp Phe Trp
   325 330 335
   Asp Ser Arg Arg Trp Gln Arg Glu Phe Glu Ser Lys Gln Val Leu Val
   340 345 350
   Met Thr Ala Gln Ile Leu Leu Asn Ile Leu Arg His Ser Ile Ile Arg
   355 360 365
   Met Glu Thr Ile Asp Leu Leu Ile Leu Asp Glu Cys His His Ala Val
   370 375 380
   Lys Lys His Pro Tyr Ser Leu Val Met Ser Glu Phe Tyr His Thr Thr
   385 390 395 400
   Pro Lys Asp Lys Arg Pro Ala Ile Phe Gly Met Thr Ala Ser Pro Val
   405 410 415
   Asn Leu Lys Gly Val Ser Ser Gln Val Asp Cys Ala Ile Lys Ile Arg
   420 425 430
   Asn Leu Glu Thr Lys Leu Asp Ser Thr Val Cys Thr Ile Lys Asp Arg
   435 440 445
   Lys Glu Leu Glu Lys His Val Pro Met Pro Ser Glu Ile Val Val Glu
   450 455 460
   Tyr Asp Lys Ala Ala Thr Met Trp Ser Leu His Glu Thr Ile Lys Gln
   465 470 475 480
   Met Ile Ala Ala Val Glu Glu Ala Ala Gln Ala Ser Ser Arg Lys Ser
   485 490 495
   Lys Trp Gln Phe Met Gly Ala Arg Asp Ala Gly Ala Lys Asp Glu Leu
   500 505 510
   Arg Gln Val Tyr Gly Val Ser Glu Arg Thr Glu Ser Asp Gly Ala Ala
   515 520 525
   Asn Leu Ile His Lys Leu Arg Ala Ile Asn Tyr Thr Leu Ala Glu Leu
   530 535 540
   Gly Gln Trp Cys Ala Tyr Lys Val Gly Gln Ser Phe Leu Ser Ala Leu
   545 550 555 560
   Gln Ser Asp Glu Arg Val Asn Phe Gln Val Asp Val Lys Phe Gln Glu
   565 570 575
   Ser Tyr Leu Ser Glu Val Val Ser Leu Leu Gln Cys Glu Leu Leu Glu
   580 585 590
   Gly Ala Ala Ala Glu Lys Val Ala Ala Glu Val Gly Lys Pro Glu Asn
   595 600 605
   Gly Asn Ala His Asp Glu Met Glu Glu Gly Glu Leu Pro Asp Asp Pro
   610 615 620
   Val Val Ser Gly Gly Glu His Val Asp Glu Val Ile Gly Ala Ala Val
   625 630 635 640
   Ala Asp Gly Lys Val Thr Pro Lys Val Gln Ser Leu Ile Lys Leu Leu
   645 650 655
   Leu Lys Tyr Gln His Thr Ala Asp Phe Arg Ala Ile Val Phe Val Glu
   660 665 670
   Arg Val Val Ala Ala Leu Val Leu Pro Lys Val Phe Ala Glu Leu Pro
   675 680 685
   Ser Leu Ser Phe Ile Arg Cys Ala Ser Met Ile Gly His Asn Asn Ser
   690 695 700
   Gln Glu Met Lys Ser Ser Gln Met Gln Asp Thr Ile Ser Lys Phe Arg
   705 710 715 720
   Asp Gly His Val Thr Leu Leu Val Ala Thr Ser Val Ala Glu Glu Gly
   725 730 735
   Leu Asp Ile Arg Gln Cys Asn Val Val Met Arg Phe Asp Leu Ala Lys
   740 745 750
   Thr Val Leu Ala Tyr Ile Gln Ser Arg Gly Arg Ala Arg Lys Pro Gly
   755 760 765
   Ser Asp Tyr Ile Leu Met Val Glu Arg Gly Asn Val Ser His Ala Ala
   770 775 780
   Phe Leu Arg Asn Ala Arg Asn Ser Glu Glu Thr Leu Arg Lys Glu Ala
   785 790 795 800
   Ile Glu Arg Thr Asp Leu Ser His Leu Lys Asp Thr Ser Arg Leu Ile
   805 810 815
   Ser Ile Asp Ala Val Pro Gly Thr Val Tyr Lys Val Glu Ala Thr Gly
   820 825 830
   Ala Met Val Ser Leu Asn Ser Ala Val Gly Leu Val His Phe Tyr Cys
   835 840 845
   Ser Gln Leu Pro Gly Asp Arg Tyr Ala Ile Leu Arg Pro Glu Phe Ser
   850 855 860
   Met Glu Lys His Glu Lys Pro Gly Gly His Thr Glu Tyr Ser Cys Arg
   865 870 875 880
   Leu Gln Leu Pro Cys Asn Ala Pro Phe Glu Ile Leu Glu Gly Pro Val
   885 890 895
   Cys Ser Ser Met Arg Leu Ala Gln Gln Ala Val Cys Leu Ala Ala Cys
   900 905 910
   Lys Lys Leu His Glu Met Gly Ala Phe Thr Asp Met Leu Leu Pro Asp
   915 920 925
   Lys Gly Ser Gly Gln Asp Ala Glu Lys Ala Asp Gln Asp Asp Glu Gly
   930 935 940
   Glu Pro Val Pro Gly Thr Ala Arg His Arg Glu Phe Tyr Pro Glu Gly
   945 950 955 960
   Val Ala Asp Val Leu Lys Gly Glu Trp Val Ser Ser Gly Lys Glu Val
   965 970 975
   Cys Glu Ser Ser Lys Leu Phe His Leu Tyr Met Tyr Asn Val Arg Cys
   980 985 990
   Val Asp Phe Gly Ser Ser Lys Asp Pro Phe Leu Ser Glu Val Ser Glu
   995 1000 1005
   Phe Ala Ile Leu Phe Gly Asn Glu Leu Asp Ala Glu Val Leu Ser Met
   1010 1015 1020
   Ser Met Asp Leu Tyr Val Ala Arg Ala Met Ile Thr Lys Ala Ser Leu
   1025 1030 1035 1040
   Ala Phe Lys Gly Ser Leu Asp Ile Thr Glu Asn Gln Leu Ser Ser Leu
   1045 1050 1055
   Lys Lys Phe His Val Arg Leu Met Ser Ile Val Leu Asp Val Asp Val
   1060 1065 1070
   Glu Pro Ser Thr Thr Pro Trp Asp Pro Ala Lys Ala Tyr Leu Phe Val
   1075 1080 1085
   Pro Val Thr Asp Asn Thr Ser Met Glu Pro Ile Lys Gly Ile Asn Trp
   1090 1095 1100
   Glu Leu Val Glu Lys Ile Thr Lys Thr Thr Ala Trp Asp Asn Pro Leu
   1105 1110 1115 1120
   Gln Arg Ala Arg Pro Asp Val Tyr Leu Gly Thr Asn Glu Arg Thr Leu
   1125 1130 1135
   Gly Gly Asp Arg Arg Glu Tyr Gly Phe Gly Lys Leu Arg His Asn Ile
   1140 1145 1150
   Val Phe Gly Gln Lys Ser His Pro Thr Tyr Gly Ile Arg Gly Ala Val
   1155 1160 1165
   Ala Ser Phe Asp Val Val Arg Ala Ser Gly Leu Leu Pro Val Arg Asp
   1170 1175 1180
   Ala Phe Glu Lys Glu Val Glu Glu Asp Leu Ser Lys Gly Lys Leu Met
   1185 1190 1195 1200
   Met Ala Asp Gly Cys Met Val Ala Glu Asp Leu Ile Gly Lys Ile Val
   1205 1210 1215
   Thr Ala Ala His Ser Gly Lys Arg Phe Tyr Val Asp Ser Ile Cys Tyr
   1220 1225 1230
   Asp Met Ser Ala Glu Thr Ser Phe Pro Arg Lys Glu Gly Tyr Leu Gly
   1235 1240 1245
   Pro Leu Glu Tyr Asn Thr Tyr Ala Asp Tyr Tyr Lys Gln Lys Tyr Gly
   1250 1255 1260
   Val Asp Leu Asn Cys Lys Gln Gln Pro Leu Ile Lys Gly Arg Gly Val
   1265 1270 1275 1280
   Ser Tyr Cys Lys Asn Leu Leu Ser Pro Arg Phe Glu Gln Ser Gly Glu
   1285 1290 1295
   Ser Glu Thr Val Leu Asp Lys Thr Tyr Tyr Val Phe Leu Pro Pro Glu
   1300 1305 1310
   Leu Cys Val Val His Pro Leu Ser Gly Ser Leu Ile Arg Gly Ala Gln
   1315 1320 1325
   Arg Leu Pro Ser Ile Met Arg Arg Val Glu Ser Met Leu Leu Ala Val
   1330 1335 1340
   Gln Leu Lys Asn Leu Ile Ser Tyr Pro Ile Pro Thr Ser Lys Ile Leu
   1345 1350 1355 1360
   Glu Ala Leu Thr Ala Ala Ser Cys Gln Glu Thr Phe Cys Tyr Glu Arg
   1365 1370 1375
   Ala Glu Leu Leu Gly Asp Ala Tyr Leu Lys Trp Val Val Ser Arg Phe
   1380 1385 1390
   Leu Phe Leu Lys Tyr Pro Gln Lys His Glu Gly Gln Leu Thr Arg Met
   1395 1400 1405
   Arg Gln Gln Met Val Ser Asn Met Val Leu Tyr Gln Phe Ala Leu Val
   1410 1415 1420
   Lys Gly Leu Gln Ser Tyr Ile Gln Ala Asp Arg Phe Ala Pro Ser Arg
   1425 1430 1435 1440
   Trp Ser Ala Pro Gly Val Pro Pro Val Phe Asp Glu Asp Thr Lys Asp
   1445 1450 1455
   Gly Gly Ser Ser Phe Phe Asp Glu Glu Gln Lys Pro Val Ser Glu Glu
   1460 1465 1470
   Asn Ser Asp Val Phe Glu Asp Gly Glu Met Glu Asp Gly Glu Leu Glu
   1475 1480 1485
   Gly Asp Leu Ser Ser Tyr Arg Val Leu Ser Ser Lys Thr Leu Ala Asp
   1490 1495 1500
   Val Val Glu Ala Leu Ile Gly Val Tyr Tyr Val Glu Gly Gly Lys Ile
   1505 1510 1515 1520
   Ala Ala Asn His Leu Met Lys Trp Ile Gly Ile His Val Glu Asp Asp
   1525 1530 1535
   Pro Asp Glu Val Asp Gly Thr Leu Lys Asn Val Asn Val Pro Glu Ser
   1540 1545 1550
   Val Leu Lys Ser Ile Asp Phe Val Gly Leu Glu Arg Ala Leu Lys Tyr
   1555 1560 1565
   Glu Phe Lys Glu Lys Gly Leu Leu Val Glu Ala Ile Thr His Ala Ser
   1570 1575 1580
   Arg Pro Ser Ser Gly Val Ser Cys Tyr Gln Arg Leu Glu Phe Val Gly
   1585 1590 1595 1600
   Asp Ala Val Leu Asp His Leu Ile Thr Arg His Leu Phe Phe Thr Tyr
   1605 1610 1615
   Thr Ser Leu Pro Pro Gly Arg Leu Thr Asp Leu Arg Ala Ala Ala Val
   1620 1625 1630
   Asn Asn Glu Asn Phe Ala Arg Val Ala Val Lys His Lys Leu His Leu
   1635 1640 1645
   Tyr Leu Arg His Gly Ser Ser Ala Leu Glu Lys Gln Ile Arg Glu Phe
   1650 1655 1660
   Val Lys Glu Val Gln Thr Glu Ser Ser Lys Pro Gly Phe Asn Ser Phe
   1665 1670 1675 1680
   Gly Leu Gly Asp Cys Lys Ala Pro Lys Val Leu Gly Asp Ile Val Glu
   1685 1690 1695
   Ser Ile Ala Gly Ala Ile Phe Leu Asp Ser Gly Lys Asp Thr Thr Ala
   1700 1705 1710
   Ala Trp Lys Val Phe Gln Pro Leu Leu Gln Pro Met Val Thr Pro Glu
   1715 1720 1725
   Thr Leu Pro Met His Pro Val Arg Glu Leu Gln Glu Arg Cys Gln Gln
   1730 1735 1740
   Gln Ala Glu Gly Leu Glu Tyr Lys Ala Ser Arg Ser Gly Asn Thr Ala
   1745 1750 1755 1760
   Thr Val Glu Val Phe Ile Asp Gly Val Gln Val Gly Val Ala Gln Asn
   1765 1770 1775
   Pro Gln Lys Lys Met Ala Gln Lys Leu Ala Ala Arg Asn Ala Leu Ala
   1780 1785 1790
   Ala Leu Lys Glu Lys Glu Ile Ala Glu Ser Lys Glu Lys His Ile Asn
   1795 1800 1805
   Asn Gly Asn Ala Gly Glu Asp Gln Gly Glu Asn Glu Asn Gly Asn Lys
   1810 1815 1820
   Lys Asn Gly His Gln Pro Phe Thr Arg Gln Thr Leu Asn Asp Ile Cys
   1825 1830 1835 1840
   Leu Arg Lys Asn Trp Pro Met Pro Ser Tyr Arg Cys Val Lys Glu Gly
   1845 1850 1855
   Gly Pro Ala His Ala Lys Arg Phe Thr Phe Gly Val Arg Val Asn Thr
   1860 1865 1870
   Ser Asp Arg Gly Trp Thr Asp Glu Cys Ile Gly Glu Pro Met Pro Ser
   1875 1880 1885
   Val Lys Lys Ala Lys Asp Ser Ala Ala Val Leu Leu Leu Glu Leu Leu
   1890 1895 1900
   Asn Lys Thr Phe Ser
   1905

## Description

This application claims the benefit of U.S. Provisional Patent Application Serial No. 60/38,316, filed June 9, 1999.

This invention was developed with government funding by the National Science Foundation, Grant No. IBN-9728239. The U.S. Government may have certain rights.

### FIELD OF THE INVENTION

The invention relates to short integuments 1 nucleic acids and proteins, and to plants having altered phenotypes when transformed with short integuments 1 nucleic acids.

### BACKGROUND OF THE INVENTION

According to recent estimates, the global demand for crop plants such as rice, wheat, and maize should increase by 40% by 2020. It is thought that classical plant breeding technology, which led to the green revolution in the late 1960s, will contribute less and less to meet this increasing demand, whereas plant genetic engineering will contribute increasingly more. An important thrust area in plant genetic engineering is the identification and use of genes implicated in asexual production of seeds, or "apomixis." Apomixis is thought to be an agronomically desirable trait that should enable seed companies and farmers to lock-in a favorable combination of genes for maximum grain yield without having to lose the gene combination in the next sexual generation. Genes for apomixis have not yet been identified. It is thought that genes that are generally important for very early embryo/seed development may be important for apomixis. A second important thrust is the production of early flowering varieties of plants such that breeding time can be reduced.

The evolution of flowering plants may have entailed a modification of primitive leaf or leaf-like structures that contained naked ovules on their surfaces, to specify floral organs that ultimately evolved to surround the ovules (Herr, "The Origin of the Ovule," Am. J. Bot. 82:547-564 (1995); Stebbins, Flowering Plants: Evolution Above the Species Level. Cambridge, MA: Harvard University Press, pp. 199-245). This view of angiosperm evolution predicts that the genetic regulatory network that controls ovule development should be interlaced with that which triggers flowering. Ovule, as the precursor of seed, is the link to the next generation. Genetic regulatory pathways that are important for early vegetative development of the embryo inside the ovule, for late reproductive development leading to the production of ovules, and for morphogenesis of the haploid female gametophyte, are crucial areas of investigation which can lead to enhanced agricultural practices.

Several genes important for ovule development have been identified in *Arabidopsis thaliana* (Reiser et al., "The Ovule and the Embryo Sac," The Plant Cell 5:1291-1301 (1993)). *BELL1,* a so-called cadastral gene that encodes a homeodomain protein (Reiser et al., "The *BELL1* Gene Encodes a Homeodomain Protein Involved in Pattern Formation in the Arabidopsis Ovule Primordium," Cell 83, 735-742 (1995)), controls the expression of the floral organ identity gene *AG* within the ovule and thereby controls morphogenesis of ovule integuments (Modrusan et al., "Homeotic Transformation of Ovules into Carpel-Like Structures in *Arabidopsis,"* The Plant Cell 6:333-349 (1994); Ray et al., "The *Arabidopsis* Floral Homeotic Gene BELL (*BEL1*) Controls Ovule Development Through Negative Regulation of AGAMOUS (*AG*) Gene," Proc. Natl. Acad. Sci. USA 91:5761-5765 (1994)). *SUPERMAN,* another cadastral gene that restricts the spatial expression pattern of the floral organ identity gene *AP3* (Sakai et al., "Role of *SUPERMAN* in Maintaining *Arabidopsis* Floral Whorl Boundaries," Nature 378:199-203 (1995)), is important in ovule integument development (Gaiser et al., "The Arabidopsis *SUPERMAN* Gene Mediates Asymmetric Growth of the Outer Integument of Ovules," The Plant Cell 7:333-345 (1995)). The organ identity gene *AP2* is also known to control ovule morphogenesis (Modrusan et al., "Homeotic Transformation of Ovules into Carpel-Like Structures in *Arabidopsis*," The Plant Cell 6:333-349 (1994)). By contrast, no known meristem identity or flowering control gene had, until now, been demonstrated to have a role in ovule development.

A gene termed SHORT INTEGUMENTS1 (*SIN1*), genetically detected in the model plant *Arabidopsis thaliana* by mutational studies has been determined to be an important regulatory gene for plant reproductive development. The *SINI* gene is required for normal ovule development (Lang et al., "*sin1,* A Mutation Affecting Female Fertility in *Arabidopsis,* Interacts with *mod1,* its Recessive Modifier," Genetics 137:11 01 -1110 (1994); Reiser et al., "The Ovule and the Embryo Sac," The Plant Cell 5:1291-1301 (1993); Robinson-Beers et al., "Ovule Development in Wild-Type *Arabidopsis* and Two Female Sterile Mutants," Plant Cell 4:1237-1250 (1992)). The original isolate of the *sin1* mutation (*sin1-1* allele) was identified as one causing a female Sterile phenotype (Robinson-Beers et al., "Ovule Development in Wild-Type *Arabidopsis* and Two Female Sterile Mutants," Plant Cell 4:1237-1250 (1992)). Ovules of the original isolate have short integuments and a defective megagametophyte (see Reiser et al., "The Ovule and the Embryo Sac," The Plant Cell 5:1291-1301 (1993) for a review on ovule structure; Baker et al., "Interactions Among Genes Regulating Ovule Development in *Arabidopsis thaliana,"* Genetics 145:1109-1124 (1997), for a recent genetic analysis; Schneitz et al., "Dissection of Sexual Organ Ontogenesis: A Genetic Analysis of Ovule Development in *Arabidopsis thaliana,"* Development 124:1367-1376 (1997), for a summary of the known mutants affected in ovule development). It has been shown that the originally-described Sin1⁻ mutant phenotype is a result of an interaction between *sin1,* and *mod1,* its recessive modifier (Lang et al., "*sin1,* A Mutation Affecting Female Fertility in *Arabidopsis,* Interacts with *mod1,* Its Recessive Modifier," Genetics 137:1101-1110 (1994)), and that *mod1* is *erecta,* a mutation in a putative serine-threonine receptor protein kinase gene. The *sin1-1* or *sin1-2* mutation acting alone causes a defect in the coordination of growth of the two sheets of cells of the inner and outer integuments. All other originally described effects on the ovule, such as the lack of outer integument cell expansion and arrest of the megagametophyte, are due to secondary genetic interactions with *erecta.* There are several prospective protein phosphorylation sites within the SIN1 protein, and these might be substrates of protein kinases, such as the ERECTA product (Torii et al., "The Arabidopsis *ERECTA* Gene Encodes a Putative Protein Kinase with Extracellular Leucine-Rich Repeats," Plant Cell 8:735-746 (1996)).

In plants homozygous for the weaker *sin1-2* mutant allele, approximately 40% of all ovules in any flower mature into seeds. But these seeds frequently contain embryos arrested at different stages of development, some of which germinate to produce abnormal seedlings. Genetic analysis shows that the maternal expression of the *SIN1* gene is necessary for embryo development (Ray et al., "Maternal Effects of the *Short Integument* Mutation on Embryo Development in *Arabidopsis,"* Dev. Biol. 180:365-369 (1996)).

Not only does this gene function in the formation of seeds, *SIN1* is the only identified plant gene whose maternal expression is important for pattern formation in the zygotic embryo (Ray et al., "Maternal Effects of the *Short Integument* Mutation on Embryo Development in *Arabidopsis,"* Dev. Biol. 180:365-369 (1996)). Both *sin1-1* and *sin1-2* alleles have the maternal-effect embryonic lethality phenotype (Ray et al., "Maternal Effects of the *Short Integument* Mutation on Embryo Development in *Arabidopsis,"* Dev. Biol. 180:365-369 (1996)). The wild type *SIN1* allele when transmitted through the pollen is unable to rescue the deleterious effects on embryogenesis of a homozygous maternal *sin1-2* mutation. Ray et al. have shown that a wild type allele of *SIN1* in the endosperm cannot rescue the maternal-effect of *sin1-2* (Ray et al., "Maternal Effects of the *Short Integument* Mutation on Embryo Development in *Arabidopsis,"* Dev. Biol. 180:365-369 (1996)). This is the first demonstration of a maternal effect embryonic pattern formation gene in a plant.

In *Arabidopsis thaliana,* meristem development progresses through at least three distinct phases: from vegetative (V) through inflorescence (I) to the floral (F) mode, a process known as the "V → I → F switch." It has been shown that the *sin1* mutation causes a defect in the V → I → F switch. *SIN1* is needed for the expression of the early flowering phenotype imparted by a TERMINAL FLOWER1 (*tfl1*) mutation, and *tfl1 sin1* double mutants do not produce pollen. Furthermore, *sin1-1* allele enhances the effect of an *APETALA1 (ap1)* mutation. Thus, *SIN1* represents a genetic connection between ovule development and control of flowering.

In addition, the function of *SIN1* gene is important for controlling the time to flower, another important agronomic factor because the timing of seed production depends on the flowering time. Ray et al. have shown by genetic analysis that *SIN1* gene regulates the activity of a master switch gene, LEAFY (*LFY*) that controls flowering time in *Arabidopsis thaliana*. The LEAFY gene from *Arabidopsis thaliana* was shown to accelerate the flowering time of aspen (an economically important timber plant) from many years to a few months. Additionally, *sin1* mutants are late flowering (Ray et al., "SHORT INTEGUMENT(sin1), A Gene Required For Ovule Development in *Arabidopsis,* Also Controls Flowering Time," Development 122, 2631-2638 (1996)) due to the production of an excess of vegetative leaves and lateral inflorescence axes before producing the floral primordia, which suggests a role of *SIN1* in meristem fate determination. The ability to improve crop plant production through genetic engineering requires the identification and manipulation of previously unidentified genes that control developmentally important plant processes, including ovule development and flowering in plants.

The present invention is directed to overcoming the deficiencies in the prior art.

### SUMMARY OF THE INVENTION

The present invention relates to an isolated nucleic acid molecule encoding a short integuments1 protein.

The present invention also relates to an isolated short integuments1 protein.

The present invention also relates to a method of regulating flowering in plants that involves transducing a plant with a DNA molecule encoding a short integuments1 protein under conditions effective to regulate flowering in the plant.

The present invention also relates to a method of increasing fertility in plants that involves transducing a plant with a DNA molecule encoding a short integuments1 protein under conditions effective to increase fertility.

The present invention also relates to a method of increasing fecundity in plants that involves transducing a plant with a DNA molecule encoding a short integuments1 protein under conditions effective to increase fecundity.

The present invention also relates to a method of decreasing fertility in plants that involves transducing a plant with a DNA molecule encoding a short integuments1 protein mutated to cause disruption of the DNA molecule under conditions effective to decrease fertility.

The present invention also relates to an expression vector containing a DNA molecule encoding a short integuments1 protein, and plant cells, plant seeds and transgenic plants transformed with a DNA molecule encoding a short integuments1 protein.

It is expected that elucidation of post-transcriptional regulation in plants will contribute significantly to the ability to control plant production through biotechnology. However, very little is currently understood about mechanisms of post-transcriptional controls, especially in plant reproduction. This invention overcomes this and other deficiencies in the art, as the *SIN1* gene and its encoded protein, which play a vital role in fertility, seed production and flowering time control in plants, provide the agronomist with important tools for engineering the expression of genes involved in seed/embryo development and flowering time.

### BRIEF DESCRIPTION OF THE DRAWINGS

Figure 1 is a map of the chromosomal region overlapping *SIN1* and the functional domains of the predicted SIN1 protein.
Figure 2 is a diagram of the BLAST derived homologies of the SIN 1 protein.

### DETAILED DESCRIPTION OF THE INVENTION

The present invention relates to an isolated nucleic acid molecule encoding a short integuments1 (SIN1) protein.

One example of the nucleic acid molecule of the present invention is the *SIN1* cDNA molecule, isolated from *Arabidopsis thaliana,* which has a nucleotide sequence corresponding to SEQ. ID. No. 1 as follows:

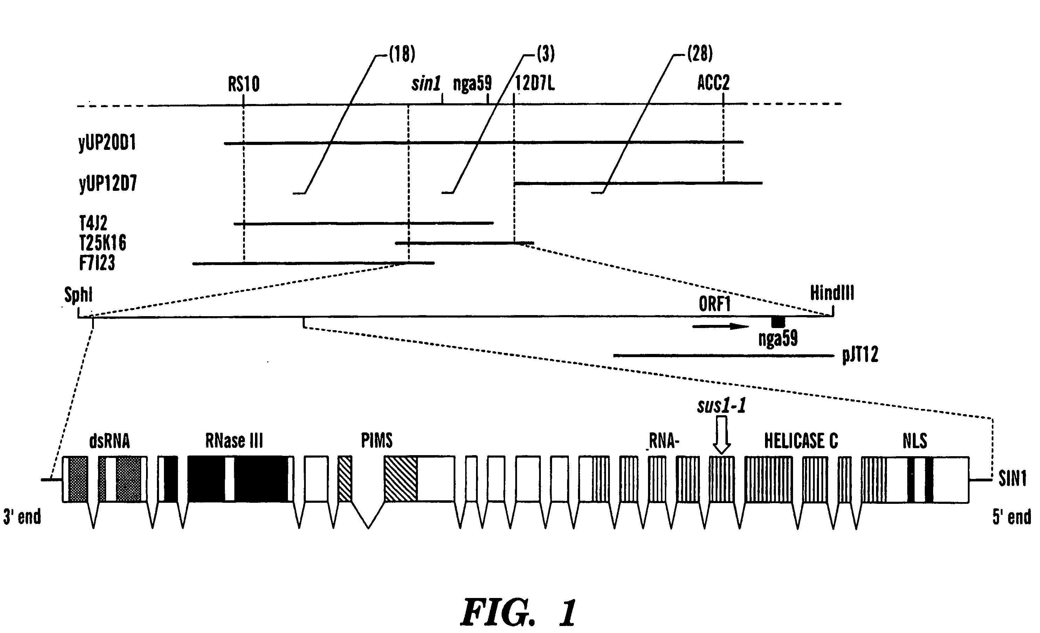

The isolated cDNA has a 5727 bp open reading frame (ORF), a 374 bp 5'-untranslated region (UTR), a 74 bp 3'-UTR and nine adenines at the 3'-end likely to be from the poly-A tail. The cDNA sequence confirmed the presence of 19 introns and 20 exons. A map of the chromosomal region overlapping *SIN1* is shown in Figure 1. RS10, nga59, 12D7L and ACC2 are DNA sequence markers. Numbers within brackets are numbers of cross-overs between La-er and Columbia chromosomes. yUP20D1 and yUP12D7 are YAC clones; T4J2, T25K16 and F7I23 are BAC clones. The lower portion of the diagram shows intron-exon boundaries of *SINI* gene. The arrow above the *sus1-1* shows that site of insertion of the linked T-DNA in *sus1-1.* That the open reading frame corresponds to *SIN1* gene is substantiated by the findings that the *sus1, sin1-1,* and *sin1-2* mutant phenotypes are traceable to DNA mutations in the *SIN1* gene. The *sus1* mutation is due to DNA insertion within the 5^{th} exon of the *SIN1* gene. The *sin1-1* and *sin1-2* phenotypes are the result of single-base pair changes, in exon 3 and exon 4, respectively. A single C to T transition in *sin1-1* and a T to A transversion in *sin1-2* reading frames were detected.

Also suitable as an isolated nucleic acid molecule according to the present invention is a nucleic acid which has a nucleotide sequence that is at least 55% similar to the nucleotide sequence of SEQ. ID. No. 1 by basic BLAST using default parameters analysis. Also suitable as an isolated nucleic acid molecule according to the present invention is an isolated nucleic acid molecule encoding a short integuments1 protein, wherein the nucleic acid hybridizes to the nucleotide sequence of SEQ. ID. No. 1 under stringent conditions characterized by a hybridization buffer comprising 0.9M sodium citrate buffer at a temperature of 45°C.

The nucleotide sequence of SEQ. ID. NO. 1 encodes a protein having an amino acid sequence corresponding to SEQ. ID. No. 2, as follows:

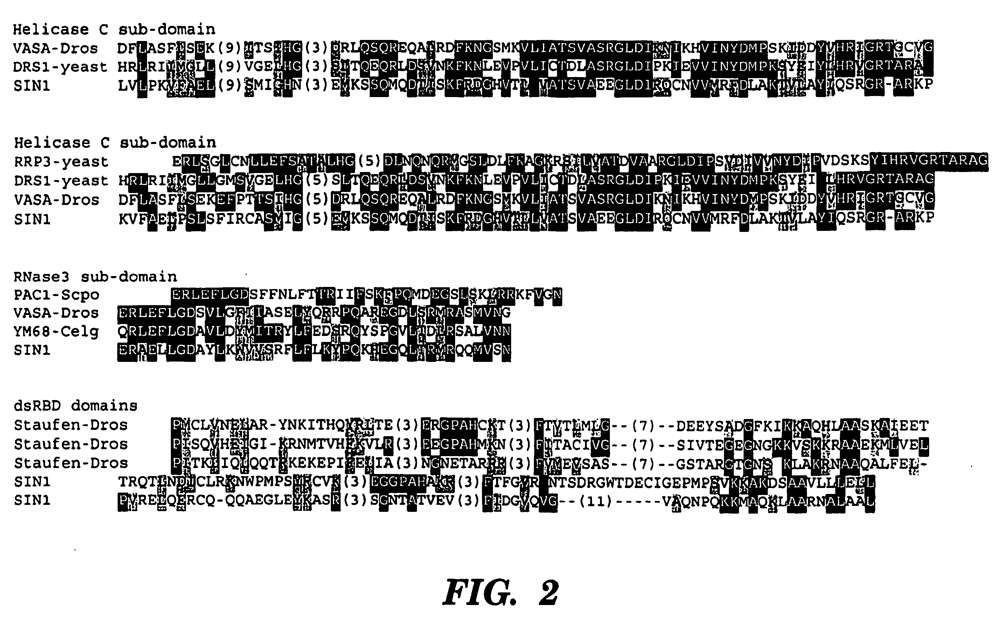

Analysis of this protein revealed a domain structure highly suggestive of an RNA helicase (Company et al., ''Requirement of the RNA Helicase-Like Protein PRP22 for Release of Messenger RNA from Spliceosomes," Nature 349:487-493 (1991); Linder et al., "Birth of the D-E-A-D Box," Nature 337:121-122 (1989); Luking et al., "The Protein Family of RNA Helicases," Crit. Rev. Biochem. Mol. Biol. 33:259-296 (1998); Martins et al., "Mutational Analysis of Vaccinia Virus Nucleoside Triphosphate Phosphohydrolase I, a DNA-Dependent ATPase of the DExH Box Family," Journal of Virology 73:1302-1308 (1999), which are hereby incorporated by reference), of which Drosophila maternal effect gene *Vasa* is a representative (Rongo et al., "Germplasm Assembly and Germ Cell Migration in Drosophila," Cold Spring Harb. Symp. Quant. Biol. 62:1-11 (1997), which is hereby incorporated by reference). Shown in the lower portion of Figure 1 is the arrangement of functional motifs of the predicted SIN1 protein: a bipartite N-terminal nuclear localization signal (NLS), an RNA helicase C domain, two RNase III catalytic domains, a PIMS (for PIWI Middle domain-SHORT INTEGUMENTS1, PIWI being a family of important plant developmental proteins) motif, and two C-terminal repeats of a dsRNA binding domain. A BLAST search yielded numerous high homology strikes of these domains, as shown in Figure 2. Each of the three functional domains is strongly conserved within its own family. For example, the helicase C motif shows strong similarity, among others, to yeast *RRP3, DRS1* and fly *Vasa* products, RNase3 domains to pombe *PAC1* or worm K12H4.8 (YM68), and dsRBD domains to Drosophila *Staufen* products.

Fragments of the above protein are also encompassed by the present invention. Suitable fragments can be produced by several means. In the first, subclones of the gene encoding the protein of the present invention are produced by conventional molecular genetic manipulation by subcloning gene fragments. The subclones then are expressed *in vitro* or *in vivo* in bacterial cells to yield a smaller protein or peptide.

In another approach, based on knowledge of the primary structure of the protein of the present invention, fragments of the gene of the present invention may be synthesized by using the PCR technique together with specific sets of primers chosen to represent particular portions of the protein. These then would be cloned into an appropriate vector for increased expression of an accessory peptide or protein.

Chemical synthesis can also be used to make suitable fragments. Such a synthesis is carried out using known amino acid sequences for the protein of the present invention. These fragments can then be separated by conventional procedures (e.g., chromatography, SDS-PAGE) and used in the methods of the present invention.

Variants may also (or alternatively) be prepared by, for example, the deletion or addition of amino acids that have minimal influence on the properties, secondary structure, and hydropathic nature of the polypeptide. For example, a polypeptide may be conjugated to a signal (or leader) sequence at the N-terminal end of the protein which co-translationally or post-translationally directs transfer of the protein. The polypeptide may also be conjugated to a linker or other sequence for ease of synthesis, purification, or identification of the polypeptide.

The present invention also relates to an expression vector containing a DNA molecule encoding a short integuments 1 protein. The nucleic acid molecule of the present invention may be inserted into any of the many available expression vectors and cell systems using reagents that are well known in the art. In preparing a DNA vector for expression, the various DNA sequences may normally be inserted or substituted into a bacterial plasmid. Any convenient plasmid may be employed, which will be characterized by having a bacterial replication system, a marker which allows for selection in a bacterium and generally one or more unique, conveniently located restriction sites. Numerous plasmids, referred to as transformation vectors, are available for plant transformation. The selection of a vector will depend on the preferred transformation technique and target species for transformation. A variety of vectors are available for stable transformation using *Agrobacterium tumefaciens*, *a* soilborne bacterium that causes crown gall. Crown gall are characterized by tumors or galls that develop on the lower stem and main roots of the infected plant. These tumors are due to the transfer and incorporation of part of the bacterium plasmid DNA into the plant chromosomal DNA. This transfer DNA (T-DNA) is expressed along with the normal genes of the plant cell. The plasmid DNA, pTI, or Ti-DNA, for "tumor inducing plasmid," contains the *vir* genes necessary for movement of the T-DNA into the plant. The T-DNA carries genes that encode proteins involved in the biosynthesis of plant regulatory factors, and bacterial nutrients (opines). The T-DNA is delimited by two 25 bp imperfect direct repeat sequences called the "border sequences." By removing the oncogene and opine genes, and replacing them with a gene of interest, it is possible to transfer foreign DNA into the plant without the formation of tumors or the multiplication of *Agrobacterium tumefaciens.* Fraley, et al., "Expression of Bacterial Genes in Plant Cells," Proc. Nat'l Acad. Sci.*,* 80:4803-4807 (1983). which is hereby incorporated by reference.

Further improvement of this technique led to the development of the binary vector system. Bevan, M., "Binary *Agrobacterium* Vectors for Plant Transformation," Nucleic Acids Res. 12:8711-8721 (1984), which is hereby incorporated by reference. In this system, all the T-DNA sequences (including the borders) are removed from the pTi, and a second vector containing T-DNA is introduced into *Agrobacterium tumefaciens.* This second vector has the advantage of being replicable in *E. coli* as well as *A. tumefaciens,* and contains a multiclonal site that facilitates the cloning of a transgene. An example of a commonly used vector is pBin19. Frisch, et al., "Complete Sequence of the Binary Vector Bin19," Plant Molec. Biol. 27:405-409 (1995), which is hereby incorporated by reference. Any appropriate vectors now known or later described for genetic transformation are suitable for use with the present invention.

U.S. Patent No. 4,237,224 issued to Cohen and Boyer, which is hereby incorporated by reference, describes the production of expression systems in the form of recombinant plasmids using restriction enzyme cleavage and ligation with DNA ligase. These recombinant plasmids are then introduced by means of transformation and replicated in unicellular cultures including procaryotic organisms and eucaryotic cells grown in tissue culture.

In one aspect of the present invention, the nucleic acid molecule of the present invention is incorporated into an appropriate vector in the sense direction, such that the open reading frame is properly oriented for the expression of the encoded protein under control of a promoter of choice.

Certain "control elements" or "regulatory sequences" are also incorporated into the vector-construct. Those non-translated regions of the vector, promoters, 5' and 3' untranslated regions-which interact with host cellular proteins to carry out transcription and translation. Such elements may vary in their strength and specificity. Depending on the vector system and host utilized, any number of suitable transcription and translation elements, including constitutive and inducible promoters, may be used.

A constitutive promoter is a promoter that directs expression of a gene throughout the development and life of an organism. Examples of some constitutive promoters that are widely used for inducing expression of transgenes include the nopoline synthase (NOS) gene promoter, from *Agrobacterium tumefaciens,* (U.S. Patent 5034322 to Rogers et al., which is hereby incorporated by reference), the cauliflower mosaic virus (CaMv) 35S and 19S promoters (U.S. Patent No. 5,352,605 to Fraley et al., which is hereby incorporated by reference), those derived from any of the several actin genes, which are known to be expressed in most cells types (U.S. Patent No. 6,002,068 to Privalle et al., which is hereby incorporated by reference), and the ubiquitin promoter, which is a gene product known to accumulate in many cell types.

An inducible promoter is a promoter that is capable of directly or indirectly activating transcription of one or more DNA sequences or genes in response to an inducer. In the absence of an inducer, the DNA sequences or genes will not be transcribed. The inducer can be a chemical agent, such as a metabolite, growth regulator, herbicide or phenolic compound, or a physiological stress directly imposed upon the plant such as cold, heat, salt, toxins, or through the action of a pathogen or disease agent such as a virus or fungus. A plant cell containing an inducible promoter may be exposed to an inducer by externally applying the inducer to the cell or plant such as by spraying, watering, heating, or by exposure to the operative pathogen. An example of an appropriate inducible promoter for use in the present invention is a glucocorticoid-inducible promoter (Schena et al., " A Steroid-Inducible Gene Expression System for Plant Cells," Proc. Natl. Acad. Sci. 88:10421-5 (1991), which is hereby incorporated by reference). Expression of the SIN1 protein is induced in the plants transformed with the *SIN1* gene when the transgenic plants are brought into contact with nanomolar concentrations of a glucocorticoid, or by contact with dexamethasone, a glucocorticoid analog. Schena et al., " A Steroid-Inducible Gene Expression System for Plant Cells," Proc. Natl. Acad. Sci. USA 88:10421-5 (1991); Aoyama et al., "A Glucocorticoid-Mediated Transcriptional Induction System in Transgenic Plants," Plant J. 11: 605-612 (1997), and McNellis et al., "Glucocorticoid-Inducible Expression of a Bacterial Avirulence Gene in Transgenic Arabidopsis Induces Hypersensitive Cell Death, Plant J. 14(2):247-57 (1998), which are hereby incorporated by reference. In addition, inducible promoters include promoters that function in a tissue specific manner to regulate the gene of interest within selected tissues of the plant. Examples of such tissue specific promoters include seed, flower, or root specific promoters as are well known in the field (U.S. Patent No. 5,750,385 to Shewmaker et al., which is hereby incorporated by reference).

The DNA construct of the present invention also includes an operable 3' regulatory region, selected from among those which are capable of providing correct transcription termination and polyadenylation of mRNA for expression in the host cell of choice, operably linked to a DNA molecule which encodes for a protein of choice. A number of 3' regulatory regions are known to be operable in plants. Exemplary 3' regulatory regions include, without limitation, the nopaline synthase 3' regulatory region (Fraley, et al., "Expression of Bacterial Genes in Plant Cells," Proc. Nat'l Acad. Sci. USA 80:4803-4807 (1983), which is hereby incorporated by reference) and the cauliflower mosaic virus 3' regulatory region (Odell, et al., "Identification of DNA Sequences Required for Activity of the Cauliflower Mosaic Virus 35S Promoter," Nature 313(6005):810-812 (1985), which is hereby incorporated by reference). Virtually any 3' regulatory region known to be operable in plants would suffice for proper expression of the coding sequence of the DNA construct of the present invention.

The vector of choice, promoter, and an appropriate 3' regulatory region can be ligated together to produce the plasmid of the present invention using well known molecular cloning techniques as described in Sambrook et al., Molecular Cloning: A Laboratory Manual, Second Edition, Cold Spring Harbor Press, NY (1989), and Ausubel, F. M. et al. (1989) Current Protocols in Molecular Biology, John Wiley & Sons, New York, N.Y., which are hereby incorporated by reference.

Once the DNA construct of the present invention has been prepared, it is ready to be incorporated into a host cell. Recombinant molecules can be introduced into cells via transformation, particularly transduction, conjugation, mobilization, or electroporation. The DNA sequences are cloned into the host cell using standard cloning procedures known in the art, as described by Sambrook et al., Molecular Cloning: A Laboratory Manual, Second Edition, Cold Springs Laboratory, Cold Springs Harbor, New York (1989), which is hereby incorporated by reference. Suitable host cells include, but are not limited to, bacteria, virus, yeast, mammalian cells, insect, plant, and the like. Preferably the host cells are either a bacterial cell or a plant cell.

Accordingly, another aspect of the present invention relates to a method of making a recombinant cell. Basically, this method is carried out by transforming a plant cell with a DNA construct of the present invention under conditions effective to yield transcription of the DNA molecule in the plant cell. Preferably, the DNA construct of the present invention is stably inserted into the genome of the recombinant plant cell as a result of the transformation.

One approach to transforming plant cells with a DNA construct of the present invention is particle bombardment (also known as biolistic transformation) of the host cell. This can be accomplished in one of several ways. The first involves propelling inert or biologically active particles at cells. This technique is disclosed in U.S. Patent Nos. 4,945,050, 5,036,006, and 5,100,792, all to Sanford, et al., which are hereby incorporated by reference. Generally, this procedure involves propelling inert or biologically active particles at the cells under conditions effective to penetrate the outer surface of the cell and to be incorporated within the interior thereof. When inert particles are utilized, the vector can be introduced into the cell by coating the particles with the vector containing the heterologous DNA. Alternatively, the target cell can be surrounded by the vector so that the vector is carried into the cell by the wake of the particle. Biologically active particles (e.g., dried bacterial cells containing the vector and heterologous DNA) can also be propelled into plant cells. Other variations of particle bombardment, now known or hereafter developed, can also be used.

Transient expression in protoplasts allows quantitative studies of gene expression since the population of cells is very high (on the order of 10⁶). To deliver DNA inside protoplasts, several methodologies have been proposed, but the most common are electroporation (Fromm et al., "Expression of Genes Transferred Into Monocot and Dicot Plants by Electroporation," Proc. Natl. Acad. Sci. USA 82:5824-5828 (1985), which is hereby incorporated by reference) and polyethylene glycol (PEG) mediated DNA uptake (Krens et al., "In Vitro Transformation of Plant Protoplasts with Ti-Plasmid DNA," Nature 296:72-74 (1982), which is hereby incorporated by reference). During electroporation, the DNA is introduced into the cell by means of a reversible change in the permeability of the cell membrane due to exposure to an electric field. PEG transformation introduces the DNA by changing the elasticity of the membranes. Unlike electroporation, PEG transformation does not require any special equipment and transformation efficiencies can be equally high. Another appropriate method of introducing the gene construct of the present invention into a host cell is fusion of protoplasts with other entities, either minicells, cells, lysosomes, or other fusible lipid-surfaced bodies that contain the chimeric gene. Fraley, et al., Proc. Natl. Acad. Sci. USA, 79:1859-63 (1982), which is hereby incorporated by reference.

Stable transformants are preferable for the methods of the present invention. An appropriate method of stably introducing the DNA construct into plant cells is to infect a plant cell with *Agrobacterium tumefaciens* or *Agrobacterium rhizogenes* previously transformed with the DNA construct. Under appropriate conditions known in the art, the transformed plant cells are grown to form shoots or roots, and develop further into plants. In one embodiment of the present invention stable transformants are generated using *Agrobacterium* using the "dipping" method, a modification of the vacuum infiltration method as described in Bent et al., "Floral Dip: A Simplified Method for Agrobacterium-Mediated Transformation of Arabidopsis thaliana," Plant J. 16:735-43 (1998), which is hereby incorporated by reference.

Plant tissues suitable for transformation include, but are not limited to, floral buds, leaf tissue, root tissue, meristems, zygotic and somatic embryos, megaspores, and anthers.

After transformation, the transformed plant cells can be selected and regenerated. Preferably, transformed cells are first identified using a selection marker simultaneously introduced into the host cells along with the DNA construct of the present invention. The most widely used reporter gene for gene fusion experiments has been *uid*A, a gene from *Escherichia coli* that encodes the β-glucuronidase protein, also known as GUS. Jefferson et al., "GUS Fusions: β Glucuronidase as a Sensitive and Versatile Gene Fusion Marker in Higher Plants," EMBO Journal 6:3901-3907 (1987), which is hereby incorporated by reference. GUS is a 68.2 kd protein that acts as a tetramer in its native form. It does not require cofactors or special ionic conditions, although it can be inhibited by divalent cations like Cu²⁺ or Zn²⁺. GUS is active in the presence of thiol reducing agents like β-mercaptoethanol or dithiothreitol (DTT).

In order to evaluate GUS activity, several substrates are available. The most commonly used are 5 bromo-4 chloro-3 indolyl glucuronide (X-Gluc) and 4 methyl-umbelliferyl-glucuronide (MUG). The reaction with X-Gluc generates a blue color that is useful in histochemical detection of the gene activity. For quantification purposes, MUG is preferred, because the umbelliferyl radical emits fluorescence under UV stimulation, thus providing better sensitivity and easy measurement by fluorometry (Jefferson et al., "GUS Fusions: β Glucuronidase as a Sensitive and Versatile Gene Fusion Marker in Higher Plants," EMBO Journal 6:3901-3907 (1987), which is hereby incorporated by reference). Other suitable selection markers include, without limitation, markers encoding for antibiotic resistance, such as the nptII gene which confers kanamycin resistance (Fraley, et al., Proc. Natl. Acad. Sci. USA, 80:4803-4807 (1983), which is hereby incorporated by reference) and the dhfr gene, which confers resistance to methotrexate (Bourouis et al., EMBO J. 2:1099-1104 (1983), which is hereby incorporated by reference). A number of antibiotic-resistance markers are known in the art and others are continually being identified. Any known antibiotic-resistance marker can be used to transform and select transformed host cells in accordance with the present invention. Cells or tissues are grown on a selection medium containing an antibiotic, whereby generally only those transformants expressing the antibiotic resistance marker continue to grow. Similarly, enzymes providing for production of a compound identifiable by luminescence, such as luciferase, are useful. The selection marker employed will depend on the target species; for certain target species, different antibiotics, herbicide, or biosynthesis selection markers are preferred.

Once a recombinant plant cell or tissue has been obtained, it is possible to regenerate a full-grown plant therefrom. Means for regeneration vary from species to species of plants, but generally a suspension of transformed protoplasts or a petri plate containing transformed explants is first provided. Callus tissue is formed and shoots may be induced from callus and subsequently rooted. Alternatively, embryo formation can be induced in the callus tissue. These embryos germinate as natural embryos to form plants. The culture media will generally contain various amino acids and hormones, such as auxin and cytokinins. It is also advantageous to add glutamic acid and proline to the medium, especially for such species as corn and alfalfa. Efficient regeneration will depend on the medium, on the genotype, and on the history of the culture. If these three variables are controlled, then regeneration is usually reproducible and repeatable.

Plant regeneration from cultured protoplasts is described in Evans, et al., Handbook of Plant Cell Cultures. Vol. 1: (MacMillan Publishing Co., New York, 1983); and Vasil I.R. (ed.), Cell Culture and Somatic Cell Genetics of Plants, Acad. Press, Orlando, Vol. I, 1984, and Vol. III (1986), which are hereby incorporated by reference.

It is known that practically all plants can be regenerated from cultured cells or tissues, including but not limited to, all major species of rice, wheat, barley, rye, cotton, sunflower, peanut, com, potato, sweet potato, bean, pea, chicory, lettuce, endive, cabbage, cauliflower, broccoli, turnip, radish, spinach, onion, garlic, eggplant, pepper, celery, carrot, squash, pumpkin, zucchini, cucumber, apple, pear, melon, strawberry, grape, raspberry, pineapple, soybean, tobacco, tomato, sorghum, sugarcane, and non-fruit bearing trees such as poplar, rubber. Paulownia, pine, and elm.

After the DNA construct is stably incorporated in transgenic plants, it can be transferred to other plants by sexual crossing or by preparing cultivars. With respect to sexual crossing, any of a number of standard breeding techniques can be used depending upon the species to be crossed. Cultivars can be propagated in accord with common agricultural procedures known to those in the field. Alternatively, transgenic seeds are recovered from the transgenic plants. The seeds can then be planted in the soil and cultivated using conventional procedures to produce transgenic plants.

Since loss of function (*Sin1* mutation) delays flowering, a gain of function, for example, by overexpression of *Sin1* gene, should promote early flowering. Accordingly, another aspect of the present invention relates to a method of increasing fertility in plants by transforming plants with the nucleic acid of the present invention. Fertility can be functionally (albeit simplistically) defined as the onset of reproductive maturity. By reducing the time from vegetative to floral stage in plants, overall breeding time can be reduced. Thus, the nucleic acid molecule of the present invention, as a regulator of flowering time, can be used to accelerate flowering in plants. This involves transforming plants with the nucleic acid of the present invention in an expression vector as described above, operably linked to an inducible promoter, such as the glucocorticoid inducible promoter. Transgenic plants in which an inducible promoter is present are treated with the suitable inducing agent (e.g., dexamethasone for the glucocorticoid inducible promoter) to induce flowering. Inducing SIN1 protein expression earlier in the development of the plant than normal accelerates early flowering, such that breeding time can be reduced. In addition, induction of flowering eliminates dependence upon external factors for flowering such as temperature and light (Coupland G., "Genetic and Environmental Control of Flowering Time in *Arabidopsis*," Mol. Gen. Genet. 242:81-89 (1995), which is hereby incorporated by reference), which are beyond the control of the average farmer. Early flowering plant lines may be especially useful for cultivation in short daylight environments.

In another aspect of the present invention, the fecundity of plants can be increased by overexpression of the nucleic acid of the present invention, under control of a constitutive promoter. Fecundity relates to reproductive maturity in combination with the total number of seeds a mature plant can produce. Thus, decreasing the time to flowering with expression of the protein of the present invention is one factor of increased fecundity, as it increases time spent in the adult phase. The other factor, seed development, is also related to expression of the protein of the present invention, as this protein, when maternally expressed, appears to coordinate the expression of zygotic pattern formation in the embryo. In this aspect of the present invention, the nucleic acid of the present invention is inserted into an expression vector, as described above, operably linked to a constitutive promoter, for example, the CaMV35S promoter. Increased expression of the protein of the present invention, which functions both in the formation of seeds and in the mother plant in embryo formation, can result in increased fecundity.

The present invention also relates to a method of decreasing fertility in plants. Because it may be commercially desirable to produce sterile female progeny, or plants with low expression of the protein of the present invention, transgenic plants can be produced in which the expression of this protein is down-regulated, or even entirely "switched off." In one aspect of the present invention, the nucleic acid of the present invention is replaced in the above-described expression vector by an antisense nucleic acid molecule which is complementary to the nucleic acid of the present invention or a fragment thereof. Antisense technology is commonplace to those skilled in the art, and the preparation of a vector and transgenic plants containing an antisense nucleic acid would be followed as described above. Transgenic plants are produced as described above, which exhibit a phenotype deficient in the nucleic acid of the present invention.

In another aspect of the present invention, the silencing of the constitutive *SIN1* gene involves the use of double-stranded RNA ("dsRNA") interference ("RNAi"), a procedure which has recently been shown to induce potent and specific post-translational gene silencing in many organisms. See Bosher et al., "RNA Interference: Genetic Wand and Genetic Watchdog," Nat Cell Biol 2:E31-6 (2000); Tavernarakis et al., "Heritable and Inducible Genetic Interference by Double-Stranded RNA encoded by Transgenes," Nat Genetics 24:180-3 (2000), which are hereby incorporated by reference. To construct transformation vectors that produce RNAs capable of duplex formation, two nucleic acid sequences according to the present invention, one in the sense and the other in the antisense orientation, are operably linked, and placed under the control of a strong viral promoter, such as CaMV 35S. The construct is introduced into the genome of *Arabidopsis thaliana* by *Agrobacterium-mediated* transformation (Chuang et al., "Specific and Heritable Genetic Interference by Double-Stranded RNA in Arabidopsis thaliana," Proc. Natl. Acad. Sci. USA 97:4985-90 (2000), which is hereby incorporated by reference), causing specific and heritable genetic interference, as evidenced by *SINI* deficient phenotype.

In another aspect of the present invention, plant lines containing insertional mutations are produced, disrupting the endogenous *SIN1* gene and thereby creating a SFN1 protein deficient plant with decreased fertility. This is accomplished by making use of well-characterized plant transposons such as the maize *Activator* ("Ac") and *Dissociation* ("Ds") family of transposable elements. The family is comprised of the autonomous element Ac, and the nonautonomous Ds element. Ds elements are not capable of autonomous transposition, but can be trans-activated to transpose by Ac. Hehl et al., "Induced Transposition of *Ds* by a Stable *Ac* in Crosses of Transgenic Tobacco Plants," Mol. Gen. Genet. 217:53-59 (1989), which is hereby incorporated by reference. Thus, transposable elements, such as Ac/Ds of maize, can be operably linked to the nucleic acid of the present invention, transferred to other plants to generate a relatively small number if anchor plants (such as 500), and then to produce a much larger number of secondary insertional-mutant plant lines. The Ac/Ds system has been improved by the use of enhancer- and gene-trap plasmids (Sundaresan et al., "Patterns of Gene Action in Plant Development Revealed by Enhancer Trap and Gene Trap Transposable Elements," Genes & Develop. 9:1797-1810 (1995), which is hereby incorporated by reference), which allow disrupted genes with no phenotype to be detected by expression of a reporter gene (such as *Gus*). After insertion of the mutant genes, plants are screened using marker genes and appropriate crosses made to produce stable mutant plant lines. Sundaresan et al., "Patterns of Gene Action in Plant Development Revealed by Enhancer Trap and Gene Trap Transposable Elements," Genes & Develop. 9:1797-1810 (1995), which is hereby incorporated by reference.

In another aspect of the present invention, the point mutations identified herein which result in *SIN1* deficient phenotypes *sus1*, *sin1-1*, and *sin1-2* can be prepared and used in the construct of the present invention to create transgenic plants and seeds carrying these point mutation alleles. The *sus1* mutation is predicted to delete most of the functional domains of the SIN1 protein. The *sin1-1* mutation produces a 415-proline to serine change in the protein; the *sin1-2* produces a 431-isoleucine to lysine change within the C-terminus helicase domain. Molecular modeling indicates that these two mutations perturb the RNA binding face of the DEHX box of the helicase C domain. Homozygous *sin1-1* or *sin1-2* mutation in *Arabidopsis* causes female sterility due to two separate phenotypic defects, and *sin1* mutants are late flowering. The allelic DNA can be synthetically produced, according to methods known to those in the art, or by inserting the above disclosed point mutations in the nucleic acid of the present invention, thereby creating plants with decreased fertility and decreased/late flowering.

In various aspects of the present invention the *SIN1* gene is either up- or down-regulated, or turned off entirely. In order to ascertain the increase or decrease in SIN1 protein expression resulting from genetic manipulation, measurement of the production of the SIN1 protein in plant tissues is carried out following transformation. Western blot, or any similar method of protein detection is appropriate, using either polyclonal or monoclonal antibodies to the protein of the present invention. Polyclonal antibodies can be produced by procedures well-known to those skilled in the art, such as those disclosed in E. Harlow, et al, editors Antibodies: A Laboratory Manual (1988), which is hereby incorporated by reference. The preparation of monoclonal antibodies, as well as Fab and *F(ab*'*)*2 fragments, also useful in protein detection methods, can be produced by various commonly used methods, such as those described in Goding, Monoclonal Antibodies: Principles and Practice, pp. 98-118, New York: Academic Press (1983), which is hereby incorporated by reference.

Although preferred embodiments have been depicted and described in detail herein, it will be apparent to those skilled in the relevant art that various modifications, additions, substitutions, and the like can be made without departing from the spirit of the invention and these are therefore considered to be within the scope of the invention as defined in the claims which follow.

## Claims

1. An isolated nucleic acid molecule encoding a short integuments1 protein, wherein the nucleic acid molecule either: 1) has a nucleotide sequence of SEQ ID No. 1 or 2) encodes a protein having an amino acid sequence of SEQ ID No. 2.

2. An isolated nucleic acid molecule according to claim 1, wherein the nucleic acid molecule encodes a protein having an amino acid sequence of SEQ. ID. No. 2.

3. An isolated nucleic acid molecule according to claim 1, wherein the nucleic acid has a nucleotide sequence of SEQ. ID. No. 1.

4. An antisense nucleic acid molecule encoding a nucleic acid sequence which is complementary to the nucleic acid molecule according to claim 1.

5. An expression vector comprising a transcriptional and translational regulatory DNA operably linked to the nucleic acid molecule according to claim 1.

6. An expression vector according to claim 5, wherein the nucleic acid molecule is in proper sense orientation and correct reading frame.

7. A host cell transduced with the nucleic acid molecule according to claim 1.

8. A host cell according to claim 7, wherein the cell is selected from a group consisting of a bacterial cell, a virus, a yeast cell, and a plant cell.

9. A transgenic plant transduced with the nucleic acid according to claim 1.

10. A transgenic plant seed transduced with the nucleic acid according to claim 1.

11. A method of increasing flowering in plants comprising:
transforming the plant with the nucleic acid molecule according to claim 1 under conditions effective to increase flowering in the plant.

12. A method of increasing fertility in plants comprising:
transforming the plant with the nucleic acid molecule according to claim 1 under conditions effective to increase fertility in the plant.

13. A method of increasing fecundity of a plant comprising:
transforming the plant with the nucleic acid molecule according to claim 1 under conditions effective to increase fecundity of the plant.

14. A method of decreasing fertility in a plant comprising:
transforming a plant with a nucleic acid molecule that either: 1) has a nucleotide sequence of SEQ ID No. 1 or 2) encodes a protein having an amino acid sequence of SEQ ID No. 2, wherein the nucleic acid molecule of 1) or 2) has a nucleotide mutation **characterised by** either a) a single C to T transition resulting in an amino acid change of 415-proline of SEQ ID No. 2 to serine or b) a single T to A transversion resulting in an amino acid change of 431-isoleucine of SEQ ID No. 2 to lysine under conditions effective to decrease fertility in the plant.

## Patentansprüche

1. Ein ein Protein kurzer Integumente1 codierendes isoliertes Nukleinsäuremolekül, bei welchem das Nukleinsäuremolekül: 1) eine Nukleotidensequenz von ID. SEQ. Nr. 1 hat, oder 2) ein Protein mit einer Aminosäuresequenz von ID. SEQ. Nr. 2 codiert.

2. Ein isoliertes Nukleinsäuremolekül gemäss Anspruch 1, bei welchem das Nukleinsäuremolekül ein Protein mit einer Aminsäuresequenz von ID. SEQ. Nr. 2 codiert.

3. Ein isoliertes Nukleinsäuremolekül gemäss Anspruch 1, bei welchem die Nukleinsäure eine Nukleotidensequenz von ID. SEQ. Nr. 1 hat.

4. Ein eine Nukleinsäuresequenz codierendes Antisense-Nukleinsäuremolekül, welches komplementär ist zum Nukleinsäuremolekül gemäss Anspruch 1.

5. Ein ein die Transkription und die Übersetzung regulierendes DNA enthaltender Expressionsvektor, welcher operativ an das Nukleinsäuremolekül gemäss Anspruch 1 gebunden ist.

6. Ein Expressionsvektor gemäss Anspruch 5, bei welchem sich das Nukleinsäuremolekül in der geeigneten Sense-Richtung und im korrekten Leserahmen befindet.

7. Eine mit dem Nukleinsäuremolekül gemäss Anspruch 1 transduzierte Wirtszelle.

8. Eine Wirtszelle gemäss Anspruch 7, bei welcher die Zelle aus einer aus einer Bakterienzelle, einem Virus, einer Hefezelle und einer Pflanzenzelle zusammengesetzten Gruppe ausgewählt wird.

9. Eine genmanipulierte, mit der Nukleinsäure gemäss Anspruch 1 transduzierte Pflanze.

10. Ein Samen einer mit der Nukleinsäure gemäss Anspruch 1 transduzierten genmanipulierten Pflanze.

11. Ein Verfahren zur Steigerung der Blüte bei Pflanzen, welches beinhaltet: die Transformation der Pflanze mit dem Nukleinsäuremolekül gemäss Anspruch 1 unter zur Steigerung der Blüte der Pflanze wirksamen Bedingungen.

12. Ein Verfahren zur Steigerung der Fruchtbarkeit bei Pflanzen, welches beinhaltet: die Transformation der Pflanze mit dem Nukleinsäuremolekül gemäss Anspruch 1 unter zur Steigerung der Fruchtbarkeit der Pflanze wirksamen Bedingungen.

13. Ein Verfahren zur Steigerung der Fekundität einer Pflanze, welches beinhaltet: die Transformation der Pflanze mit dem Nukleinsäuremolekül gemäss Anspruch 1 unter zur Steigerung der Fekundität der Pflanze wirksamen Bedingungen.

14. Ein Verfahren zur Verminderung der Fruchtbarkeit bei einer Pflanze, welches beinhaltet: die Transformation der Pflanze mit einem Nukleinsäuremolekül, welches: 1) eine Nukleotidensequenz von ID. SEQ. Nr. 1 hat, oder 2) ein Protein mit einer Aminosäuresequenz von ID. SEQ. Nr. 2 codiert, wobei das Nukleinsäuremolekül von 1) oder 2) eine Nukleotidenmutation aufweist, **gekennzeichnet durch** a) einen einfachen Übergang von C zu T, der zu einem Wechsel bei der Aminosäure von 415-Prolin der ID. SEQ. Nr. 2 zu Serin führt, oder **durch** b) eine einfache Transversion von T zu A, die zu einem Wechsel bei der Aminsäure von 431- Isoleucin der ID. SEQ. Nr. 2 zu Lisin führt, unter zur Verminderung der Fruchtbarkeit der Pflanze wirksamen Bedingungen.

## Revendications

1. Une molécule d'acide nucléique isolée qui codifie une protéine de tégument 1 court, dans laquelle la molécule d'acide nucléique : 1) a une séquence de nucléotides de SEQ. ID. n° 1, ou bien 2) codifie une protéine avec une séquence d'acides aminés de SEQ. ID. n° 2.

2. Une molécule d'acide nucléique isolée selon la revendication 1, dans laquelle la molécule d'acide nucléique codifie une protéine avec une séquence d'acides aminés de SEQ. ID. n° 2.

3. Une molécule d'acide nucléique isolée selon la revendication 1, dans laquelle l'acide nucléique a une séquence de nucléotides de SEQ. ID. n° 1.

4. Une molécule d'acide nucléique antisens qui codifie une séquence d'acide nucléique qui est complémentaire à la molécule d'acide nucléique selon la revendication 1.

5. Un vecteur d'expression qui comprend un ADN régulateur de la transcription et de la traduction uni opérationnellement à la molécule d'acide nucléique selon la revendication 1.

6. Un vecteur d'expression selon la revendication 5, dans lequel la molécule d'acide nucléique est dans l'orientation sens adéquate et dans le cadre de lecture correct.

7. Une cellule hôte transduite avec la molécule d'acide nucléique selon la revendication 1.

8. Une cellule hôte selon la revendication 7, dans laquelle la cellule est choisie dans un groupe constitué par une cellule bactérienne, un virus, une cellule de levure et une cellule végétale.

9. Une plante transgénique transduite avec l'acide nucléique selon la revendication 1.

10. Une graine d'une plante transgénique transduite avec l'acide nucléique selon la revendication 1.

11. Un procédé pour augmenter la floraison chez les plantes qui comprend : transformer la plante avec la molécule d'acide nucléique selon la revendication 1 dans des conditions efficaces pour augmenter la floraison de la plante.

12. Un procédé pour augmenter la fertilité chez les plantes qui comprend : transformer la plante avec la molécule d'acide nucléique selon la revendication 1 dans des conditions efficaces pour augmenter la fertilité de la plante.

13. Un procédé pour augmenter la fécondité d'une plante qui comprend : transformer la plante avec la molécule d'acide nucléique selon la revendication 1 dans des conditions efficaces pour augmenter la fécondité de la plante.

14. Un procédé pour réduire la fertilité d'une plante qui comprend : transformer une plante avec une molécule d'acide nucléique qui : 1) a une séquence de nucléotides de SEQ. ID. n° 1, ou bien 2) codifie une protéine avec une séquence d'acides aminés de SEQ. ID. n° 2, dans laquelle la molécule d'acide nucléique de 1) ou de 2) a une mutation de nucléotides **caractérisée par** a) une transition simple de C à T qui donne comme résultat un changement de l'acide aminé de proline 415 de SEQ. ID. n° 2 en sérine, ou bien par b) une transversion simple de T en A qui donne comme résultat un changement de l'acide aminé d'isoleucine 431 de SEQ. ID. n° 2 en lysine dans des conditions efficaces pour réduire la fertilité de la plante.
